# EUROPÄISCHE PATENTSCHRIFT

(11) **EP 3 536 290 B1**
(45) Veröffentlichungstag und Bekanntmachung des Hinweises auf die Patenterteilung: **09.12.2020**
(21) Anmeldenummer: 18160558.5
(22) Anmeldetag: 07.03.2018
(51) Int. Cl.: A61F 9/02, G02C 11/00, A41D 13/11

(54) **SCHUTZBRILLE UND SCHUTZSYSTEM FÜR EINEN MENSCHLICHEN BENUTZER**
PROTECTIVE EYEWEAR AND PROTECTION SYSTEM FOR A HUMAN USER
LUNETTES PROTECTRICES ET SYSTÈME DE PROTECTION POUR UN UTILISATEUR HUMAIN

(43) Veröffentlichungstag der Anmeldung: 11.09.2019
(73) Patentinhaber: Moldex-Metric AG & Co. KG, 72141 Walddorfhäslach (DE)
(72) Erfinder: SKOV, Torben, 72124 Pliezhausen (DE); SKOV, Roman, 70197 Stuttgart (DE)
(74) Vertreter: Clarenbach, Carl-Philipp

(56) Entgegenhaltungen:
- EP-A2- 0 625 344
- DE-U1- 29 721 661
- US-A1- 2004 237 962
- US-A1- 2007 252 946
- US-A1- 2008 143 953

## Beschreibung

Die Erfindung betrifft eine Schutzbrille für einen menschlichen Benutzer, mit zumindest einer Brillenscheibe, die einen der Stirn des Benutzers zuordenbaren oberen Rand und einen einer Wange des Benutzers zuordenbaren unteren Rand aufweist, mit zumindest einer an der Brillenscheibe angeordneten Nasenauflage und mit zumindest einer Schutzlippe zur Auflage auf dem Gesicht des Benutzers.

Weiterhin betrifft die Erfindung ein Schutzsystem für einen menschlichen Benutzer, das eine Schutzbrille und eine Atemmaske aufweist, die separat ausgebildet beziehungsweise handhabbar sind.

Schutzbrillen und Schutzsysteme sind aus dem Stand der Technik bereits bekannt. Schutzbrillen dienen dazu, die Augen eines Benutzers vor Staub, Späne, Flüssigkeit, Temperatur oder anderer Produkte zu schützen, die insbesondere bei der Bearbeitung eines Werkstücks entstehen und in Richtung des Benutzers geschleudert werden können. Üblicherweise weist die Schutzbrille ein oder zwei Brillenscheiben aus einem durchsichtigen Material auf, die dem Brechungsindex einer herkömmlichen Glasscheibe aufweisen und insofern die Optik des Benutzers nicht beeinträchtigen können. Natürlich gibt es aber auch Schutzbrillen, welche einen an den Benutzer angepassten Brechungsindex der jeweiligen Brillenscheibe aufweisen.

Damit die Augen während des Tragens der Schutzbrille ausreichend Sauerstoff zur Verfügung haben und ein Beschlagen der Brillenscheiben unterbleibt, werden die Brillenscheiben üblicherweise beabstandet zu den Augen und dem Gesicht des Benutzers gehalten. Hierfür ist üblicherweise eine Nasenauflage vorgesehen, welche mit den Brillenscheiben fest verbunden ist und diese auf der Nase des Benutzers derart positioniert, dass ein Abstand zu den Augen des Benutzers beziehungsweise dem Gesicht des Benutzers verbleibt. Weil nunmehr zwischen dem Außenrand des jeweiligen Brillenglas und dem Gesicht des Benutzers die oben genannten Produkte dennoch in dem Bereich der Augen des Benutzers gelangen könnten, ist es bekannt, diesen Abstand durch eine Schutzlippe zu überbrücken.

So offenbart beispielsweise die Offenlegungsschrift EP 2 215 997 B1 eine Schutzbrille der gattungsgemäßen Art, die eine durchgehende Brillenscheibe für beide Augen des Benutzers aufweist, sowie eine Schutzlippe, die sich über den gesamten Rand beziehungsweise äußeren Umfang der Brillenscheibe erstreckt, und dadurch den Bereich zwischen Gesicht des Benutzers und Brillenscheibe vollumfänglich überdeckt, sodass die zuvor genannten Produkte nicht in den Augenbereich des Benutzers gelangen können. Damit dennoch ausreichend Sauerstoff dem Benutzer zugeführt wird, weist die Schutzlippe mehrere Öffnungen auf. Die Schutzlippe erstreckt sich dabei von dem Seitenrand der Brillenscheibe direkt in Richtung des Gesichts des Benutzers, steht also im Wesentlichen in einem rechten Winkel zu der Brillenscheibe in Richtung des Benutzers ab. Hierdurch wird die Sicht des Benutzers möglichst wenig beeinträchtigt und der Raum, welchen die Schutzbrille auf dem Gesicht des Benutzers benötigt, miniert. Hierdurch werden Tragekomfort und Schutzwirkung gleichermaßen gewährleistet.

Im Zusammenspiel mit Atemmasken kann dies jedoch zu dem Problem führen, dass zwischen Schutzbrille und Atemmaske nicht ausreichend Raum zur Verfügung steht, welcher ein bequemes/komfortables Tragen beider Elemente für den Benutzer gewährleistet. Insbesondere kann es dadurch dazu kommen, dass im Benutzungsbetrieb Atemmaske und Schutzbrille ständig oder zeitweise aneinander geraten, was für den Benutzer beispielsweise durch ein Verschieben der Schutzbrille neben Komfortbeeinträchtigungen auch dazu führen kann, dass sich die Schutzlippe vom Gesicht des Benutzers löst.

Aus der Offenlegungsschrift US 2007/0252946 A1 ist des Weiteren eine Schutzbrille bekannt, bei welcher Schutzlippen lösbar an einer Brillenscheibe der Schutzbrille befestigbar sind. Ähnliche Schutzbrillen gehen auch aus der Offenlegungsschrift US 2008/0143953 A1 sowie aus der Offenlegungsschrift EP 0 625 344 A2 hervor. Aus der Offenlegungsschrift US 2004/0237962 A1 ist außerdem eine Schutzbrille bekannt, bei welcher am unteren Rand einer Brillenscheibe eine luftdurchlässige Membran befestigbar ist.

Der Erfindung liegt daher die Aufgabe zugrunde, eine verbesserte Schutzbrille zu schaffen, welche insbesondere im Zusammenspiel mit einer Atemmaske den Benutzungskomfort verbessert bei gleichzeitiger Sicherstellung des Schutzes der Augen des Benutzers vor Schadprodukten.

Die der Erfindung zugrundeliegende Aufgabe wird durch eine Schutzbrille mit den Merkmalen des Anspruchs 1 gelöst. Diese hat den Vorteil, dass durch die vorteilhafte Ausbildung der Schutzbrille ein hoher Benutzungskomfort sowie eine hohe Betriebssicherheit gewährleistet werden, und dass die Integration einer Atemmaske in das aus Schutzbrille und Atemmaske gebildete Schutzsystem einfach und problemlos erfolgt. Vorhandener Raum wird auf dem Gesicht des Benutzers optimal ausgenutzt und Schmutzpartikel sicher daran gehindert, die Augen des Benutzers zu erreichen. Erfindungsgemäß ist hierzu vorgesehen, dass sich die Schutzlippe nur in einem unteren Rand der Brillenscheibe von der Nasenauflage aus in Richtung zumindest eines Seitenrands der Brillenscheibe und von dem unteren Rand der Brillenscheibe in Verlängerung der Brillenscheibe nach unten zur Auflage auf der Wange des Benutzers erstreckt. Dadurch, dass sich die Schutzlippe im Wesentlichen nur am unteren Rand der Brillenscheibe entlang erstreckt, ist zumindest der obere Randbereich der Brille frei von der Dichtlippe und erlaubt einen Sauerstoff- beziehungsweise Luftaustausch und verhindert ein Beschlagen der Brillenscheibe. Dadurch, dass sich die Schutzlippe vom unteren Rand der Brillenscheibe weg in Verlängerung der Brillenscheibe nach unten zur Auflage auf der Wange eines Benutzer erstreckt, und nicht, wie bisher üblich, senkrecht von der Brillenscheibe direkt in Richtung des Gesichts, entsteht eine Schutzlippe, welche die Wange des Benutzers flächig überdeckt und sich in Richtung des Bereichs erstreckt, in welchem ansonsten eine Atemmaske angeordnet wird. Die Atemmaske ist dabei optional auf der Schutzlippe platzierbar, sodass sich Schutzlippe und Atemmaske überlappen und zum einen das Gesicht und die Augen des Benutzers sicher geschützt sind, und zum anderen eine sichere Auflage der Schutzlippe auf dem Gesicht des Benutzers einerseits sowie der Atemmaske auf dem Gesicht des Benutzers andererseits, dann unter Zwischenschaltung der Schutzlippe, gesichert ist. Durch die Schutzlippe wird die Brillenscheibe nach unten verlängert bis an die Wange des Benutzers heran, sodass ein sicherer und dauerhafter Schutz des Benutzers und insbesondere der Augen des Benutzers besteht. Darüber hinaus wird durch die vorteilhafte Ausbildung der Schutzbrille erreicht, dass die Brillenscheibe besonders nahe zum Gesicht des Benutzers anordenbar ist. Weil die Schutzlippe sich nicht senkrecht von der Brillenscheibe in Richtung des Gesichts des Benutzers erstreckt, sondern in Verlängerung der Brillenscheibe nach unten, ist der Abstand zwischen Brillenscheibe und Gesicht des Benutzers minimierbar. Dies hat zum einen den Vorteil, dass beispielsweise ein Luftspalt zwischen Gesicht des Benutzers und Brillenscheibe in einem schutzlippenfreien Abschnitt minimiert ist, was die Sicherheit für den Benutzer weiter erhöht, und dass zum anderen eine Kollisionsgefahr mit einer gegebenenfalls gleichzeitig verwendeten Atemschutzmaske reduziert wird. Durch das nahe Anordnen der Brillenscheibe am Gesicht des Benutzers wird zusätzlicher Raum für die Atemschutzmaske geschaffen, sodass der Benutzer sowohl Atemschutzmaske als auch Schutzbrille komfortabel und sicher tragen kann.

Gemäß einer bevorzugten Weiterbildung der Erfindung ist vorgesehen, dass die Schutzbrille nur eine durchgehende Brillenscheibe zum Schutz beider Augen des Benutzers aufweist, und dass sich die Schutzlippe nur an einem unteren Rand der Brillenscheibe von der Nasenauflage aus in Richtung beider Seitenränder der Brillenscheibe erstreckt. Hierdurch ist eine einfache und kompakte Schutzbrille geboten, die kostengünstig herstellbar ist und aufgrund der wenigen Einzelteile auch eine einfache Handhabung gewährleistet.

Gemäß einer alternativen Ausführungsform der Erfindung weist die Schutzbrille für jedes Auge des Benutzers eine eigene Brillenscheibe auf, wobei die Nasenauflage mit beiden Brillenscheiben verbunden ist, und dass sich jeweils eine Schutzlippe nur in einem unteren Rand der Brillenscheibe von der Nasenauflage aus in Richtung des äußeren Seitenrands der jeweiligen Brillenscheibe erstreckt. Somit weist die Schutzbrille zwei Schutzlippen oder Schutzlippenteile auf, die sich jeweils von der Nasenauflage ausgehend nach außen zum Seitenrand der jeweiligen Brillenscheibe, also zum Außenrand der jeweiligen Brillenscheibe entlang des unteren Rands erstrecken. Hierdurch wird für den Benutzer derselbe Schutz geboten und es werden dieselben Vorteile erreicht, wie sie vorstehend beschrieben wurden. Je nach Herstellungsverfahren ist die Herstellung der Schutzbrille durch das Vorsehen von zwei getrennten Brillenscheiben kostengünstiger und erlaubt eine höhere Flexibilität bei der Anpassung der Schutzbrille an das Gesicht des Benutzers.

Gemäß einer bevorzugten Ausführungsform der Erfindung ist die jeweilige Schutzlippe, also die eine durchgehende mit einer durchgehenden Brillenscheibe oder die zwei Schutzlippen oder Schutzlippenteile mit den zwei Brillenscheiben, dazu ausgebildet, flächig auf der Wange des Benutzers aufzuliegen. Durch die flächige Auflage ist der Tragekomfort verbessert und die Dichtwirkung optimiert. Darüber hinaus wird dadurch eine große Auflagefläche für die Atemmaske auf der Schutzlippe geboten.

Weiterhin weist die jeweilige Schutzlippe bevorzugt in ihrer Erstreckung nach unten eine Krümmung zur flächigen Auflage auf der Wange des Benutzers auf. Durch die Krümmung ist die Form der Schutzlippe an das Gesicht angepasst und erlaubt einen bequemen und sicheren, insbesondere dichtenden Kontakt mit dem Gesicht des Benutzers.

Besonders bevorzugt ist die Brillenscheibe konvex gekrümmt und die Krümmung der jeweiligen Schutzlippe zumindest abschnittsweise konkav. Dadurch ergibt sich eine S-förmige Kontur der Schutzbrille aus Brillenscheibe und Schutzlippe. Durch diese Kontur ist eine vorteilhafte Anlage am Gesicht des Benutzers durch die Schutzlippe einerseits sowie ein vorteilhafter Schutz für die Augen des Benutzers andererseits gewährleistet.

Die jeweilige Schutzlippe ist insbesondere einstückig mit der Nasenauflage ausgebildet. Dadurch ergibt sich letztendlich eine durchgehende Schutzlippe für die Schutzbrille, unabhängig davon, ob die Schutzbrille eine oder zwei Brillenscheiben aufweist. Im Bereich der Nase wird die Schutzlippe lediglich durch die Form der Nasenauflage unterbrochen.

Besonders bevorzugt bildet die durchgehende Schutzlippe die Nasenauflage mit. Dadurch weist die Nasenauflage eine Form auf, die auch der Form der Schutzlippe entspricht. Durch die vorteilhafte Ausbildung der Schutzlippe ergibt sich eine automatische vorteilhafte Nasenauflage, die auf der vom Gesicht abgewandten Seite des Benutzers liegt. Hierdurch ergibt sich außerdem der Vorteil, dass die Schutzbrille besonders nahe zum Gesicht des Benutzers anordenbar ist. Dadurch wird der Abstand zwischen den Brillenscheiben oder der Brillenscheibe und beispielswiese der Stirn des Benutzers auf ein Minimum reduziert, sodass das Eindringen von Schmutzpartikeln an dieser Stelle ebenfalls verhindert wird und gleichzeitig ein Luftaustausch zur Vermeidung eines Beschlagens der Brillenscheiben und/oder zur Sauerstoffversorgung der Augen gewährleistet ist.

Vorzugsweise ist die Nasenauflage zumindest im Wesentlichen auf der vom Gesicht des Benutzers abgewandten Seite der Schutzbrille angeordnet. Dies ist in diesem Fall insbesondere unabhängig davon, ob die Nasenauflage durch die Schutzlippe mitgebildet, mit dieser einstückig oder separat von dieser ausgebildet ist. Es ergibt sich dadurch der zuvor bereits genannte Vorteil bezüglich des minimierten Abstands zwischen Brillenscheibe oder Brillenscheiben und Stirn des Benutzers.

Weiterhin ist bevorzugt vorgesehen, dass die jeweilige Schutzlippe elastisch verformbar ausgebildet ist. Hierdurch ergibt sich ein hoher Tragekomfort und ein sicheres Abdichten auch bei unterschiedlichen Gesichtsformen. Weiterhin weist die jeweilige Schutzlippe bevorzugt eine zu dem vom unteren Rand der Brillenscheibe abgewandten Ende abnehmende Dicke auf. Dadurch läuft die Schutzlippe beispielsweise spitz zu und erleichtert beispielsweise das Auflegen einer Atemschutzmaske.

Gemäß einer bevorzugten Weiterbildung der Erfindung weist die jeweilige Schutzlippe eine von dem Benutzer abgewandte Maskenauflagenfläche auf, auf welcher eine Atemmaske für den Benutzer auflegbar ist. Durch die Maskenauflagefläche erstreckt sich die Schutzlippe beispielsweise weiter über die Wange des Benutzers als es für die eigentliche Dichtfunktion notwendig wäre. Dadurch wird jedoch sichergestellt, dass die Atemmaske sicher auf der Schutzlippe ablegbar ist und das vorteilhafte Zusammenwirken zwischen Atemmaske und Schutzlippe, wie vorstehend bereits erläutert, erreicht ist.

Besonders bevorzugt weist die Maskenauflagefläche eine Gleitoberfläche auf, um Relativbewegungen zwischen Atemmaske und Schutzbrille zu ermöglichen. Dazu ist die Gleitoberfläche bevorzugt auf das Material und/oder die Oberflächenstruktur der Atemmaske angepasst, um einen möglichst geringen Reibwiderstand zu gewährleisten. Hierdurch wird erreicht, dass im Betrieb bei einem Verrutschen der Atemmaske die Schutzbrille nicht mitverschoben wird. Außerdem wird dadurch erreicht, dass durch ein Bewegen der Atemmaske nicht die Dichtwirkung zwischen Schutzbrille und Gesicht des Benutzers aufgehoben wird.

Gemäß einer bevorzugten Weiterbildung der Erfindung ist die jeweilige Schutzlippe zumindest im Wesentlichen durchsichtig ausgebildet. Hierdurch verlängert die Schutzlippe die Brillenscheibe auch in ihrer Funktion, sodass der Benutzer auch durch die Schutzlippe seinen Blick nach unten wenden kann.

Erfindungsgemäß ist die jeweilige Schutzlippe an die jeweilige Brillenscheibe stoffschlüssig angeformt. Dadurch ist eine integrale Ausbildung der Schutzlippe an der Brillenscheibe gewährleistet und eine einfache Handhabung sowie eine einfache Montage geboten. Insbesondere wird dadurch vermieden, dass sich die Schutzlippe im Betrieb von der Brillenscheibe lösen kann.

Das erfindungsgemäße Schutzsystem mit den Merkmalen des Anspruchs 15 zeichnet sich durch die erfindungsgemäße Ausbildung der Schutzbrille aus. Es ergeben sich hierdurch die bereits genannten Vorteile. Gemäß einer ersten Ausführungsform sind die Atemschutzmaske und die Schutzbrille derart ausgebildet, dass die Schutzmaske bei der Verwendung zumindest bereichsweise auf der Schutzlippe der Schutzbrille aufliegt, wobei die Schutzlippe dabei bevorzugt die bereits genannte Maskenauflagefläche aufweist. Gemäß einer weiteren Ausführungsform sind Schutzbrille und Atemschutzmaske dazu ausgebildet, nebeneinander auf dem Gesicht des Benutzers zu liegen, sodass keine Überlappung/Uberschneidung erfolgt. Dadurch liegen Schutzbrille und Atemschutzmaske jeweils für sich genommen dichtend am Gesicht des Benutzers an. Aufgrund der vorteilhaften Ausbildung der Schutzbrille ist dabei ausreichend Raum für die Atemschutzmaske zur Verfügung gestellt, sodass bei einer Verschiebung der Atemschutzmaske durch den Benutzer diese nicht gegen die Schutzbrille stößt und dadurch die Dichtung beeinträchtigt, und andersherum.

Weitere Vorteile und bevorzugte Merkmale und Merkmalskombinationen ergeben sich insbesondere aus dem zuvor Beschriebenen sowie aus den Ansprüchen. Im Folgenden soll die Erfindung anhand der Zeichnung näher erläutert werden. Dazu zeigen
- Figur 1: eine Schutzbrille für einen menschlichen Benutzer in einer perspektivischen vereinfachten Darstellung,
- Figur 2: die Schutzbrille aus Figur 1 in einer ersten Schnittdarstellung und
- Figur 3: die Schutzbrille aus Figur 1 in einer zweiten Schnittdarstellung.

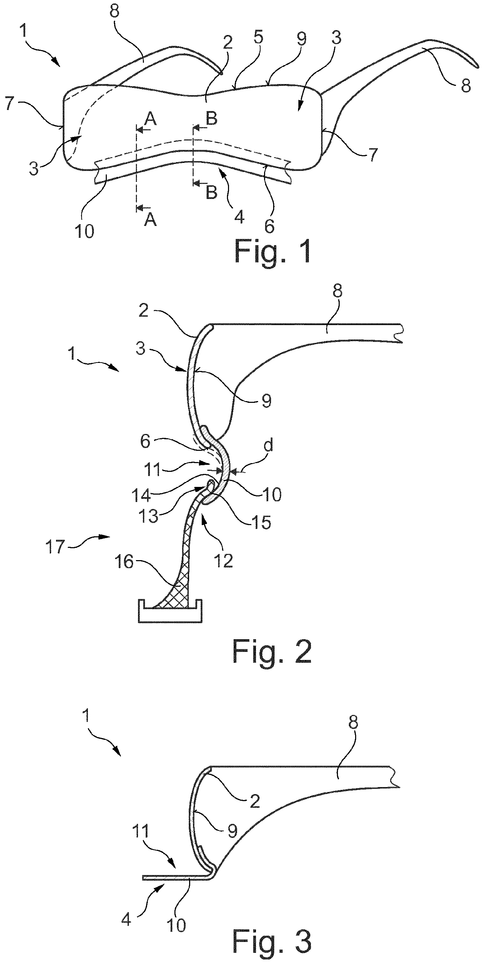

Figur 1 zeigt in einer vereinfachten perspektivischen Darstellung eine Schutzbrille 1, die zur Benutzung durch einen menschlichen Benutzer ausgebildet ist. Dazu weist die Schutzbrille 1 eine Brillenscheibe 2 auf, die dazu ausgebildet ist, beide Augen eines Benutzers zu überdecken. Die Brillenscheibe 2 weist somit zwei Brillenscheibenabschnitte 3 auf, wobei jeder Brillenscheibenabschnitt 3 jeweils einem Auge des Benutzers zuordenbar ist. Zwischen den Brillenscheibenabschnitten 3 weist die Schutzbrille 1 eine Nasenauflage 4 auf, welche zur Auflage der Schutzbrille 1 auf der Nase des Benutzers und zur Positionierung an dem Gesicht dient. Die Nasenauflage 4 wird im Weiteren näher erörtert. Die Nasenauflage 4 liegt somit mittig an der Brillenscheibe 2. Die Brillenscheibe 2 weist einen einer Stirn des Benutzers zuordenbaren oberen Rand 5, einen der Nase sowie den Wangen des Benutzers zuordenbaren unteren Rand 6, welchem die Nasenauflage 4 zugeordnet ist, sowie zwei jeweils den oberen Rand 5 mit dem unteren Rand 6 verbindende äußere Seitenränder 7 auf. Jedem der äußeren Seitenränder 7 ist außerdem ein Brillenbügel 8 zugeordnet, der sich von der Brillenscheibe 2 nach hinten beziehungsweise in Richtung des Gesichts des Benutzers erstreckt. Die Brillenbügel 8 sind somit der Rückseite 9 der Brillenscheibe 2 zugeordnet.

Weiterhin weist die Schutzbrille 1 eine Schutzlippe 10 auf, die dem unteren Rand 6 der Brillenscheibe 2 zugeordnet ist. Die Schutzlippe 10 erstreckt sich gemäß dem vorliegenden Ausführungsbeispiel nahezu über den gesamten unteren Rand 6 der Brillenscheibe 2. Dabei erstreckt sich die Schutzlippe 10 zumindest im Wesentlichen über beide Brillenscheibenabschnitte 3 bis nahe zu dem jeweiligen Seitenrand 7. Der verbleibende Abstand zum Seitenrand 7 kann je nach Ausführungsbeispiel größer oder kleiner gewählt sein, auch kann sich die Schutzlippe 10 bis zum Seitenrand 7, gegebenenfalls auch darüber hinaus, erstrecken. Dadurch, dass sich die Schutzlippe 10 auch über den mittleren Bereich der Brillenscheibe 2 erstreckt, erstreckt sich die Schutzlippe 10 über die Nasenauflage 7. Gemäß dem vorliegenden Ausführungsbeispiel ist die Schutzlippe 10 derart gestaltet, dass sie die Nasenauflage 4 bildet.

Figur 2 zeigt eine erste Schnittdarstellung der Schutzbrille 1 aus Figur 1 entlang der Linie A-A. Der Schnitt verläuft somit etwa mittig durch einen Brillenscheibenabschnitt 3. Die Schutzlippe 10 ist an dem unteren Rand 6 befestigt. Insbesondere ist die Schutzlippe 10 dazu auf die Rückseite 9 der Brillenscheibe 2 aufgespritzt, so dass eine stoffschlüssige Verbindung zwischen Schutzlippe 10 und Brillenscheibe 2 entsteht. Während die Brillenscheibe 2 selbst wie üblich durchsichtig ausgebildet ist, kann die Schutzlippe 10 auch aus einem nicht-durchsichtigen Material gefertigt sein. Gemäß dem vorliegenden Ausführungsbeispiel ist die Schutzlippe ebenfalls aus einem durchsichtigen Material gefertigt. Optional ist die Schutzlippe 10 auch aus einem nicht-durchsichtigen Material fertigbar.

Insbesondere ist die Schutzlippe 10 elastisch verformbar ausgebildet, weist jedoch im unbelasteten beziehungsweise unverformten/neutralen Zustand eine Krümmung 11 auf. Zum einen ist die Schutzlippe 10 durch den Verlauf entlang des Rands 6 an der Brillenscheibe 2 in ihrem Verlauf entlang der Brillenscheibe 2 gekrümmt. Die Krümmung 11 der Schutzlippe 10 erstreckt sich zum anderen in Schnitt gesehen von der Brillenscheibe 2 bis zu deren unteren Rand 6 nach unten. Die Schutzlippe 10 ist derart ausgebildet, dass sie zunächst dem Verlauf der Brillenscheibe 2 folgt und letztendlich die Brillenscheibe 2 an ihrem unteren Rand 6 verlängert. Während die Brillenscheibe 2 gemäß dem vorliegenden Ausführungsbeispiel in der Draufsicht gesehen konvex gekrümmt ist, ist die Schutzlippe 10 durch die Krümmung 11 konkav geformt, so dass sich aus Brillenscheibe 2 und Schutzlippe 10 eine S-Form ergibt. Durch die vorteilhafte Gestalt der Schutzlippe 10 ist diese als Auflage auf der Wange des Benutzers geeignet. Durch die Krümmung 11 ist insbesondere eine komfortable flächige Auflage der Schutzlippe 10 auf der Wange gewährleistet, die einerseits einen hohen Tragekomfort der Schutzbrille 1 für den Benutzer gewährleistet und andererseits eine sichere Dichtung zwischen Brillenscheibe 2 und Gesicht des Benutzers, welche verhindert, dass beispielsweise bei der spanenden Bearbeitung von Werkstücken Späne an die Augen des Benutzers gelangen können. Die Schutzlippe 10 ist elastisch verformbar ausgebildet, so dass sie sich an unterschiedliche Gesichtsformen vorteilhaft anpasst und in jedem Fall eine komfortable und sichere Dichtung bietet. Während gemäß dem vorliegenden Ausführungsbeispiel die Schutzlippe 10 eine durchgehend konstante Dicke d aufweist, ist gemäß einem weiteren, hier nicht dargestellten Ausführungsbeispiel vorgesehen, dass die Dicke d der Schutzlippe 10 zu dem freien von dem unteren Rand 6 der Bremsscheibe 2 abgewandten Ende 12 abnimmt. Dadurch wird die Flexibilität der Schutzlippe 10 an ihrem freien Ende erhöht und der Tragekomfort noch weiter verbessert. Dadurch, dass sich die Schutzlippe 10 in Verlängerung der Brillenscheibe 2 nach unten erstreckt, ist außerdem die Brillenscheibe 2 besonders nahe zum Gesicht des Benutzers anordenbar, wodurch erreicht wird, dass ein Luftspalt zwischen Brillenscheibe und Gesicht des Benutzers minimiert und dadurch das Eindringen von Staub oder Späne in den Bereich zwischen Brillenscheibe und Gesicht verhindert wird, und dass auf dem Gesicht des Benutzers mehr Raum für eine Atemschutzmaske zur Verfügung steht, sodass insbesondere Atemschutzmaske und Schutzbrille 1 während der Verwendung nicht gegeneinander stoßen und dadurch beispielsweise die Dichtheit von Schutzbrille 1 oder Atemschutzmaske auf dem Gesicht des Benutzers beeinträchtigen.

Darüber hinaus weist die Schutzlippe 10 optional an ihrer vom Gesicht des Benutzers 2 abgewandten Vorderseite 13 insbesondere im Bereich des freien Endes 12 eine Maskenauflagefläche 14 auf, auf welcher ein Auflagerand 15 einer hier gestrichelt dargestellten Atemschutzmaske 16 auflegbar ist. Dadurch ist eine überlappende Anordnung von Atemschutzmaske 16 und Schutzbrille 1 möglich, die zum einen den sicheren Schutz der Augen des Benutzers durch die Schutzlippe 10 gewährleistet, und zum anderen eine Relativbewegung zwischen Schutzbrille 1 und Atemschutzmaske 16 erlaubt, so dass während der Benutzung ein Verschieben der Schutzbrille 2 oder der Atemschutzmaske 16 nicht auch zu einem Verschieben der Atemschutzmaske 16 beziehungsweise der Schutzbrille 1 führt. Darüber hinaus bleibt auch bei einem Verschieben der Atemschutzmaske die Dichtung zwischen Schutzlippe 10 und Gesicht des Benutzers erhalten, wodurch die Betriebssicherheit maximiert ist. Die Schutzbrille 1 und die Atemschutzmaske 16 bilden insoweit ein Schutzsystem 17 für den menschlichen Benutzer, das einerseits einen hohen Tragekomfort und andererseits eine hohe Betriebssicherheit gewährleistet. Bei Gesichts- und insbesondere Mundbewegungen bewegt sich gegebenenfalls die Atemschutzmaske 16, um einen dichten Sitz zu gewährleisten. Durch die vorteilhafte Kombination mit der Schutzbrille 1 wird verhindert, dass sich dadurch auch die Schutzbrille 2 bewegt oder verschiebt. Die Maskenauflagefläche 14 weist insbesondere eine niedrige Rauigkeit auf, die eine geringe Reibhaftung zwischen Atemschutzmaske 16 und Schutzlippe 10 gewährleistet, so dass eine einfache Relativbewegung ermöglicht ist. Die Maskenauflagefläche 14 kann dabei eine sich vom Rest der Schutzlippe 10 verschiedenen Abschnitt der Schutzlippe 10 beziehungsweise deren Oberfläche ausbilden. Alternativ weist die Schutzlippe 10 insgesamt die gleichen Oberflächeneigenschaften wie an der Maskenauflagefläche 14 auf.

Während gemäß dem vorliegenden Ausführungsbeispiel die Schutzlippe 10 nur an der Rückseite 9 der Brillenscheibe 2 befestigt ist, ist gemäß einem weiteren Ausführungsbeispiel, das in Figur 2 durch gestrichelte Linien gezeigt ist, die Schutzlippe 10 sowohl an Rückseite 9 als auch an der vom Benutzer abgewandten Vorderseite aufgebracht, erfindungsgemäß ausgespritzt, um einen sicheren Halt der Schutzlippe 10 an der Brillenscheibe 2 auch bei höheren Belastungen zu gewährleisten.

Figur 3 zeigt eine zweite Schnittdarstellung der Schutzbrille 1 entlang der Linie B-B aus Figur 1, der sich durch die Mitte der Brillenscheibe 2 erstreckt, so dass in der Schnittdarstellung der Figur 3 auch die Nasenauflage 4 ersichtlich ist.

Im Bereich der Nasenauflage 4 erstreckt sich die Schutzlippe 10 aufgrund ihrer konkaven Krümmung 11, die im Bereich der Nasenauflage 4 auch im entspannten Zustand der Schutzlippe 10 optional stärker ausgeprägt ist als im Bereich der Brillenscheibenabschnitte 3, zumindest bei Benutzung nahezu senkrecht von der Brillenscheibe 2 nach vorne, also vom Gesicht des Benutzers weg. Dadurch bildet die Schutzlippe 10 selbst an ihrer dem Benutzer zugewandten Rückseite 18 die Nasenauflage 4. Zusätzliche Mittel zum Bereitstellen einer Nasenauflage 4 sind somit nicht notwendig. Die Form der Schutzlippe 10 aus Figur 3 kann sich einerseits durch das Aufsetzen der Schutzbrille 1 auf die Nase des Benutzers ergeben, oder bereits durch die Vorformung der Schutzlippe 10 bereitgestellt sein, um beispielsweise den Tragekomfort zu erhöhen. Damit ist die Nasenauflage 4 vorteilhafterweise einstückig mit der Schutzlippe 10 ausgebildet.

Gemäß einem alternativen, hier nicht dargestellten Ausführungsbeispiel, kann die Nasenauflage 4 auch separat zu der Schutzlippe 10 oder zu zwei Schutzlippen 10, die sich jeweils über einen der Brillenscheibenabschnitte 3 erstrecken, ausgebildet sein.

Gemäß einem weiteren Ausführungsbeispiel ist vorgesehen, dass die Brillenscheibenabschnitte von jeweils zwei separaten Brillenscheiben 2 gebildet werden, so dass jeweils eine Brillenscheibe 2 einem Auge des Benutzers zugeordnet ist. In diesem Fall werden die Brillenscheiben 2 dann beispielsweise durch die Nasenauflage 4 aneinander gehalten.

Dadurch, dass sich die Nasenauflage 4 von dem Gesicht des Benutzers weg nach vorne erstreckt, ist die jeweilige Brillenscheibe 2 besonders nahe zum Gesicht des Benutzers anordenbar, so dass beispielsweise auch der Abstand zwischen dem oberen Rand 5 der Brillenscheibe 2 und der Stirn des Benutzers minimiert wird, wodurch auch an dieser Stelle ein Schutz der Augen des Benutzers vor beispielsweise Späne gewährleistet ist.

Durch die integrierte Ausbildung von Schutzlippe 10 und Nasenauflage 4 ergibt sich eine besonders kompakte, kostengünstig herzustellende und einfach handhabbare Schutzbrille 1, die insbesondere in dem Schutzsystem 17 in Zusammenwirkung mit der Atemschutzmaske 16 die oben genannten Vorteile bietet. Durch die vorteilhafte Schutzlippe 10 ist außerdem erreichbar, dass die Brillenscheibe 2 insgesamt eine niedrigere Höhe aufweisen kann, als bei bisherigen Schutzbrillen üblich, die den Schutz am unteren Rand 6 durch eine besonders weit nach unten verlaufende Brillenscheibe 2 suchen. Dieser Rand ist jedoch bei Standardschutzbrillen komforteinschränkend und nicht an das Gesicht des Benutzers flexibel anpassbar. Durch die vorliegende vorteilhaft ausgebildete Schutzbrille 1 ist es möglich, mit einer einzigen Schutzbrille 1 verschiedensten Benutzern einen komfortablen sicheren Schutz zu bieten. Durch die schmalere Brillenscheibe 2 wird auch der Raum für die Atemschutzmaske 16 vergrößert und dadurch Kombinationen auch von unterschiedlichen Atemschutzmasken vereinfacht.

## Patentansprüche

1. Schutzbrille (1) für einen menschlichen Benutzer, mit zumindest einer Brillenscheibe (2), die einen der Stirn des Benutzers zuordenbaren oberen Rand (5) und einen einer Wange des Benutzers zuordenbaren unteren Rand (6) aufweist, mit zumindest einer an der Brillenscheibe (2) angeordneten Nasenauflage (4), und mit zumindest einer Schutzlippe (10) zur Auflage auf dem Gesicht des Benutzers, wobei sich die Schutzlippe (10) nur an dem unteren Rand (6) der Brillenscheibe (2) von der Nasenauflage (4) aus in Richtung zumindest eines äußeren Seitenrands (7) der Brillenscheibe (2) und von dem unteren Rand (6) der Brillenscheibe in Verlängerung der Brillenscheibe (2) nach unten zur Auflage auf der Wange des Benutzers erstreckt, **dadurch gekennzeichnet, dass** die Schutzlippe (10) an die Brillenscheibe (2) durch Aufspritzen auf die Brillenscheibe (2) stoffschlüssig angeformt ist.

2. Schutzbrille nach Anspruch 1, **dadurch gekennzeichnet, dass** sie nur eine durchgehende Brillenscheibe (2) zum Schutz beider Augen des Benutzers aufweist, dass sich die Schutzlippe (10) nur an dem unteren Rand der Brillenscheibe (2) von der Nasenauflage (4) aus jeweils in Richtung beider äußeren Seitenränder (7) der Brillenscheibe (2) erstreckt.

3. Schutzbrille nach Anspruch 1, **dadurch gekennzeichnet, dass** sie für jedes Auge des Benutzers eine eigene Brillenscheibe (2) aufweist, wobei die Nasenauflage (4) mit beiden Brillenscheiben (2) verbunden ist, dass sich jeweils eine Schutzlippe (10) nur an einem unteren Rand der jeweiligen Brillenscheibe von der Nasenauflage (4) aus in Richtung des äußeren Seitenrands (7) der jeweiligen Brillenscheibe (2) erstreckt.

4. Schutzbrille nach einem der vorhergehenden Ansprüche, **dadurch gekennzeichnet, dass** die jeweilige Schutzlippe (10) dazu ausgebildet ist, flächig auf der Wange des Benutzers aufzuliegen.

5. Schutzbrille nach einem der vorhergehenden Ansprüche, **dadurch gekennzeichnet, dass** die jeweilige Schutzlippe (10) in ihrer Erstreckung nach unten eine Krümmung (11) zur flächigen Auflage auf der Wange des Benutzers aufweist.

6. Schutzbrille nach einem der vorhergehenden Ansprüche, **dadurch gekennzeichnet, dass** die Brillenscheibe (2) konvex gekrümmt ist, und dass die Krümmung (11) der jeweiligen Schutzlippe (10) zumindest abschnittsweise konkav ausgebildet ist.

7. Schutzbrille nach einem der vorhergehenden Ansprüche, **dadurch gekennzeichnet, dass** die jeweilige Schutzlippe (10) einstückig mit der Nasenauflage (4) ausgebildet ist.

8. Schutzbrille nach einem der vorhergehenden Ansprüche, **dadurch gekennzeichnet, dass** die durchgehende Schutzlippe (10) die Nasenauflage (4) bildet.

9. Schutzbrille nach einem der vorhergehenden Ansprüche, **dadurch gekennzeichnet, dass** die jeweilige Schutzlippe (10) elastisch verformbar ausgebildet ist.

10. Schutzbrille nach einem der vorhergehenden Ansprüche, **dadurch gekennzeichnet, dass** die Nasenauflage (4) zumindest im Wesentlichen auf der von dem Gesicht des Benutzers abgewandten Seite der Brillenscheibe (2) angeordnet ist.

11. Schutzbrille nach einem der vorhergehenden Ansprüche, **dadurch gekennzeichnet, dass** die jeweilige Schutzlippe (10) eine von dem Benutzer abgewandte Maskenauflagefläche (14) aufweist, auf welcher eine Atemschutzmaske (16) für den Benutzer auflegbar ist.

12. Schutzbrille nach einem der vorhergehenden Ansprüche, **dadurch gekennzeichnet, dass** die Maskenauflagefläche (14) eine Gleitoberfläche aufweist, um Relativbewegungen zwischen Atemschutzmaske (16) und Schutzbrille (1) zu erleichtern.

13. Schutzbrille nach einem der vorhergehenden Ansprüche, **dadurch gekennzeichnet, dass** die jeweilige Schutzlippe (10) zumindest im Wesentlichen durchsichtig ausgebildet ist.

14. Schutzsystem (17) für einen menschlichen Benutzer, mit einer Schutzbrille (1) und mit einer Atemschutzmaske (16), die separat handhabbar sind, **gekennzeichnet durch** die Ausbildung der Schutzbrille (1) nach einem der Ansprüche 1 bis 13.

## Claims

1. Safety glasses (1) for a human user, with at least one lens (2) which has an upper rim (5) assignable to the user's forehead and a lower rim (6) assignable to a user's cheek, with at least one nosepiece (4) arranged at the lens (2) and with at least one protective lip (10) to be supported on the user's face, whereby the protective lip (10) only extends at the lower rim (6) of the lens (2) from the nosepiece (4) towards at least one outer lateral rim (7) of the lens (2) and, in prolongation of the lens (2), downwards from the lower rim (6) of the lens to be supported on the user's cheek, **characterised in that** the protective lip (10) is integrally formed on the lens (2) by spraying it onto the lens (2).

2. Safety glasses according to claim 1, **characterised in that** they only have one continuous lens (2) to protect both of the user's eyes and that the protective lip (10) only extends at the lower rim of the lens (2) from the nosepiece (4) towards each of both outer lateral rims (7) of the lens (2).

3. Safety glasses according to claim 1, **characterised in that** it has a separate lens (2) for each of the user's eyes, wherein the nosepiece (4) is connected to both lenses (2), and that in each case a protective lip (10) only extends at a lower rim of each respective lens from the nosepiece (4) towards the outer lateral rim (7) of the respective lens (2).

4. Safety glasses according to any one of the preceding claims, **characterised in that** the respective protective lip (10) is configured to be flatly supported on the user's cheek.

5. Safety glasses according to any one of the preceding claims, **characterised in that** the respective protective lip (10) has a curvature (11) in its downward extension so that it is flatly supported on the user's cheek.

6. Safety glasses according to any one of the preceding claims, **characterised in that** the lens (2) has a convex curvature and that the curvature (11) of the respective protective lip (10) is concave at least in certain sections.

7. Safety glasses according to any one of the preceding claims, **characterised in that** the respective protective lip (10) is integrally formed with the nosepiece (4).

8. Safety glasses according to any one of the preceding claims, **characterised in that** the continuous protective lip (10) forms the nosepiece (4).

9. Safety glasses according to any one of the preceding claims, **characterised in that** the respective protective lip (10) is configured such that it is elastically deformable.

10. Safety glasses according to any one of the preceding claims, **characterised in that** the nosepiece (4) is, at least essentially, arranged on the side of the lens (2) facing away from the user's face.

11. Safety glasses according to any one of the preceding claims, **characterised in that** the respective protective lip (10) has a mask support surface (14) facing away from the user, wherein a respiratory mask (16) for the user can be supported on said mask support surface (14).

12. Safety glasses according to any one of the preceding claims, **characterised in that** the mask support surface (14) has a sliding surface in order to facilitate relative movements between the respiratory mask (16) and the safety glasses (1).

13. Safety glasses according to any one of the preceding claims, **characterised in that** the respective protective lip (10) is, at least essentially, configured transparent.

14. A safety system (17) for a human user, with safety glasses (1) and with a respiratory mask (16) which can be handled separately, **characterised by** the configuration of the safety glasses (1) in accordance with any one of claims 1 to 13.

## Revendications

1. Lunettes protectrices (1) pour un utilisateur humain, avec au moins un verre de lunette (2), présentant un bord supérieur (5) qui peut être associé au front de l'utilisateur et un bord inférieur (6) qui peut être associé à une joue de l'utilisateur, avec au moins un plaquette nasale (4) disposé sur le verre de lunettes (2), et avec d'au moins une lèvre de protection (10) pour l'appui sur le visage de l'utilisateur, la lèvre de protection (10) ne s'étendant que sur le bord inférieur (6) du verre de lunette (2), à partir du plaquette nasale (4) dans la direction d'au moins un bord latéral extérieur (7) du verre de lunette (2), et à partir du bord inférieur (6) du verre de lunette en extension du verre de lunette (2) vers le bas pour l'appui sur la joue de l'utilisateur, **caractérisées en ce que** la lèvre de protection (10) est moulée sur le verre de lunette (2) en liaison matérielle au moyen de moulage par injection sur le verre de lunette (2).

2. Lunettes protectrices selon la revendication 1, **caractérisées en ce qu'**elles comprennent seulement un verre de lunette (2) continu pour la protection des deux yeux de l'utilisateur, et **en ce que** la lèvre de protection (10) se n'étend que sur le bord inférieur du verre de lunette (2), à partir du plaquette nasale (4) respectivement dans la direction des deux bords latéraux extérieurs (7) du verre de lunette (2).

3. Lunettes protectrices selon la revendication 1, **caractérisées en ce qu'**elles comprennent un verre de lunette (2) séparât pour chaque œil de l'utilisateur, le plaquette nasale (4) étant relié avec les deux verres de lunette (2), **en ce que** respectivement une lèvre de protection (10) se n'étend que sur un bord inférieur du verre de lunette respectif à partir du plaquette nasale (4) dans la direction du bord latéral extérieur (7) du verre de lunette (2) respectif.

4. Lunettes protectrices selon l'une quelconque des revendications précédentes, **caractérisées en ce que** la lèvre de protection (10) respective est configurée pour rester à plat sur la joue de l'utilisateur.

5. Lunettes protectrices selon l'une quelconque des revendications précédentes, **caractérisées en ce que** la lèvre de protection (10) respective présente, sur son extension vers le bas, une courbure (11) pour l'appui à plat sur la joue de l'utilisateur.

6. Lunettes protectrices selon l'une quelconque des revendications précédentes, **caractérisées en ce que** le verre de lunette (2) a une courbure convexe, et **en ce que** la courbure (11) de la lèvre de protection (10) respective est d'au moins dans certaines régions configurées sous forme concave.

7. Lunettes protectrices selon l'une quelconque des revendications précédentes, **caractérisées en ce que** la lèvre de protection (10) respective est formée d'un seul tenant avec le plaquette nasale (4).

8. Lunettes protectrices selon l'une quelconque des revendications précédentes, **caractérisées en ce que** la lèvre de protection (10) continue forme le plaquette nasale (4).

9. Lunettes protectrices selon l'une quelconque des revendications précédentes, **caractérisées en ce que** la lèvre de protection (10) respective est configurée pour être élastiquement déformable.

10. Lunettes protectrices selon l'une quelconque des revendications précédentes, **caractérisées en ce que** le plaquette nasale (4) est disposé d'au moins sensiblement sur le côté du verre de lunette (2) opposé au visage de l'utilisateur.

11. Lunettes protectrices selon l'une quelconque des revendications précédentes, **caractérisées en ce que** la lèvre de protection (10) respective a une surface d'appui du masque (14) opposée à l'utilisateur sur laquelle peut être disposé un masque respiratoire protecteur (16) pour l'utilisateur.

12. Lunettes protectrices selon l'une quelconque des revendications précédentes, **caractérisées en ce que** la surface d'appui du masque (14) a une surface de coulissement pour faciliter des mouvements relatifs entre le masque respiratoire protecteur (16) et les lunettes protectrices (1).

13. Lunettes protectrices selon l'une quelconque des revendications précédentes, **caractérisées en ce que** la lèvre de protection (10) respective est d'au moins sensiblement configurée sous forme transparente.

14. System protecteur (17) pour un utilisateur humain, avec des lunettes protectrices (1) et avec un masque respiratoire protecteur (16) qui peuvent être manipulés séparément, **caractérisé par** la configuration des lunettes protectrices (1) selon l'une quelconque des revendications 1 à 13.
